# EUROPEAN PATENT SPECIFICATION

(11) **EP 3 891 176 B1**
(45) Date of publication and mention of the grant of the patent: **05.06.2024**
(21) Application number: 19812797.9
(22) Date of filing: 04.12.2019
(51) Int. Cl.: C07K 14/605, C07K 7/08, C12N 15/62, C07K 14/575, C07K 7/22

(54) **DUAL AGONIST GLP-1 AND NEUROTENSIN FUSION PEPTIDE**
DOPPELAGONIST GLP-1 UND NEUROTENSINFUSIONSPEPTID
PEPTIDE DE FUSION DE GLP-1 ET DE NEUROTENSINE À DOUBLE EFFET AGONISTE

(30) Priority: 04.12.2018 EP 18210211
(43) Date of publication of application: 13.10.2021
(73) Proprietor: Holst, Birgitte, 2820 Gentofte (DK); Ratner, Cecilia, 2100 København Ø (DK)
(72) Inventor: HOLST, Birgitte, 2200 Copenhagen N (DK); RATNER, Cecilia, 2200 Copenhagen N (DK)
(74) Representative: AWA Denmark A/S
(86) International application number: PCT/EP2019/083634
(87) International publication number: WO 2020/115113

(56) References cited:
- WO-A1-2017/035432
- KAARE V. GRUNDDAL ET AL: "Neurotensin Is Coexpressed, Coreleased, and Acts Together With GLP-1 and PYY in Enteroendocrine Control of Metabolism", ENDOCRINOLOGY, vol. 157, no. 1, 1 January 2016 (2016-01-01), pages 176-194, XP055584016, US ISSN: 0013-7227, DOI: 10.1210/en.2015-1600

## Description

### Technical Field

This invention relates to the field of therapeutic peptides, i.e. to peptide fusions of Glucagon-Like Peptide-1 (GLP-1) derivatives and Neurotensin derivatives.

### Background Art

Obesity is the most prevalent nutritional disease of humans and domestic animals such as dogs and cats in affluent societies, exceeding by far the number of nutritional deficiency diseases. As alternatives to bariatric surgery, many attempts have been made to provide an appetite regulating drug for the treatment of obesity. This has resulted in drugs that act by preventing the absorption of fats by acting as lipase inhibitors, or as hypothalamic acting inhibitors of food intake such as the selective serotonin receptor 2c agonist Lorcaserin. However, adverse effects of hypothalamus activators may include effects on mood and increase in the likelihood of suicide. Glucagon-like peptide 1 is a cleavage product of the pre-proglucagon gene, and a recent indication of GLP-1 is for weight maintenance, since it acts in the appetite regulating centres of the brain. However, it also delays gastric emptying and gut motility. GLP-1 is of relevance to appetite and weight maintenance because it has actions on the gastrointestinal tract as well as the direct regulation of appetite. It also delays gastric emptying and gut motility in humans, which both contribute to regulating food intake.

WO2003/040309 discloses peptides acting as both GLP-1 receptor agonists and glucagon receptor antagonists. Among the disclosed peptides are two peptides which have been coupled to polyethyleneglycol via a C-terminal cysteine residue to decrease renal clearance. WO2004/093823 and WO2000/066629 disclose polyethylene glycated glucagon and exendin agonists, respectfully. WO2017/035432 discloses co-agonists of the GLP-1 receptor and the neuropeptide Y2 receptor, wherein the co-agonist is a fusion protein comprising the GLP-1 analog exendin-4 and PYY.

However, there is a constant need for novel treatments with greater efficacy and offering convenient and safe administration options. It is therefore an object of the present invention to provide an effective and safe therapeutic agent to reduce appetite and food intake.

### Summary of Invention

The inventors have surprisingly found that fusion peptides comprising an appetite regulating hormone peptide, e.g. glucagon like peptide 1 (GLP-1), such as amino acids 7-37 of the initial GLP-1 product (defined by sequence of 37 amino acids 1-37), and a Neurotensin (NT) like peptide target both the GLP-1 receptor (GLP-1R) and NT receptors (NTR1, NTR2, NTR3) display a synergistic effect on decrease of appetite and food intake and body weight compared to simultaneous administration of both peptides.

Accordingly, a first aspect of the present invention relates to a fusion peptide comprising a first peptide linked to a second peptide, optionally via a linker molecule, which first peptide comprises the sequence X₁-X₂-X₃-X₄-X₅-X₆-X₇-X₈-X₉ wherein:
X₁ is L-histidine, D-histidine, desamino-histidine, 2-amino-histidine, β hydroxy-histidine, homohistidine, N^{α}-acetyl-histidine, α-fluoromethyl-histidine, α-methyl-histidine, 3-pyridylalanine, 2-pyridylalanine or 4-pyridylalanine;
X₂ is A, G, V, L, I, K, S, aminoisobutyric acid (Aib), (1-aminocyclopropyl) carboxylic acid, (1 aminocyclobutyl) carboxylic acid, (1-aminocyclopentyl) carboxylic acid, (1 aminocyclohexyl) carboxylic acid, (1-aminocycloheptyl) carboxylic acid, or (1-aminocyclooctyl) carboxylic acid;
X₃ is E, D or Q;
X₄ is G or A;
X₅ is T, V, S or I;
X₆ is F or Y;
X₇ is T, or S;
X₈ is S, V, or D;
X₉ is S, D, E, N or is not present, or
the first peptide comprising the sequence laid out in SEQ ID NO: 40 or SEQ ID NO: 41 or SEQ ID NO: 42, and
which second peptide has an amino acid sequence with at least 70% identity with any one of SEQ ID NO:24 to SEQ ID NO:31, or wherein the second peptide is selected from the list consisting of:
   X₁₀-X₁₁-P-X₁₂-I-L;
   P-X₁₀-X₁₁-P-X₁₂-I-L;
   K-P-X₁₀-X₁₁-P-X₁₂-I-L;
   N-K-P-X₁₀-X₁₁-P-X₁₂-I-L;
   E-N-K-P-X₁₀-X₁₁-P-X₁₂-I-L;
   Y-E-N-K-P-X₁₀-X₁₁-P-X₁₂-I-L;
   L-Y-E-N-K-P-X₁₀-X₁₁-P-X₁₂-I-L;
   Q-L-Y-E-N-K-P-X₁₀-X₁₁-P-X₁₂-I-L; or
   E-L-Y-E-N-K-P-X₁₀-X₁₁-P-X₁₂-I-L
   wherein
   X₁₀ is R or K;
   X₁₁ is R or K; and
   X₁₂ is Y, S, C or T, and wherein the fusion peptide is a dual agonist of both a glucagon like peptide 1 receptor and a neurotensin receptor.

Thus, in one embodiment the fusion peptide comprises GLP-1 fragment [7-36] and Neurotensin peptide [1-13] forming a GLP-1/NT fusion fusion peptide.

In another embodiment, the fusion peptide comprises GLP-1 fragment [7-37] and Neurotensin peptide [1-13] forming a GLP-1/NT fusion polypeptide.

GLP-1 is a cleavage product of the pre-proglucagon gene which is expressed in the pancreas, the L-cells of the intestine, and the central nervous system. The initial GLP-1 product (1-37) is susceptible to amidation and proteolytic cleavage which gives rise to the two truncated and equipotent biologically active forms, GLP-1 (7-36) amide and GLP-1 (7-37). GLP-1 is shown as SEQ ID NO: 2. Glucagon-like peptide 1 (7-36)-amide (GLP-1) has a well-known physiological role in regulating satiety, having an important gut hormone with regulatory function in glucose metabolism and gastrointestinal secretion and metabolism; it stimulates insulin secretion in a glucose-dependant manner, stimulates insulin biosynthesis, promotes beta cell rescue and decreases glucagon secretion.

Neurotensin (NT) is a 13-amino acid peptide expressed in the brain as well as localized in specialized enteroendocrine cells of the small intestine, where it is released by fat ingestion and facilitates fatty acid translocation and smooth muscle contraction. NT is shown in SEQ ID NO: 24. It also exerts neuromodulatory functions in the central nervous system with highest levels in the hypothalamus, amygdala and nucleus accumbens. It induces a variety of effects, including food intake inhibition, analgesia, hypothermia and increased locomotor activity. It is also involved in regulation of dopamine pathways.

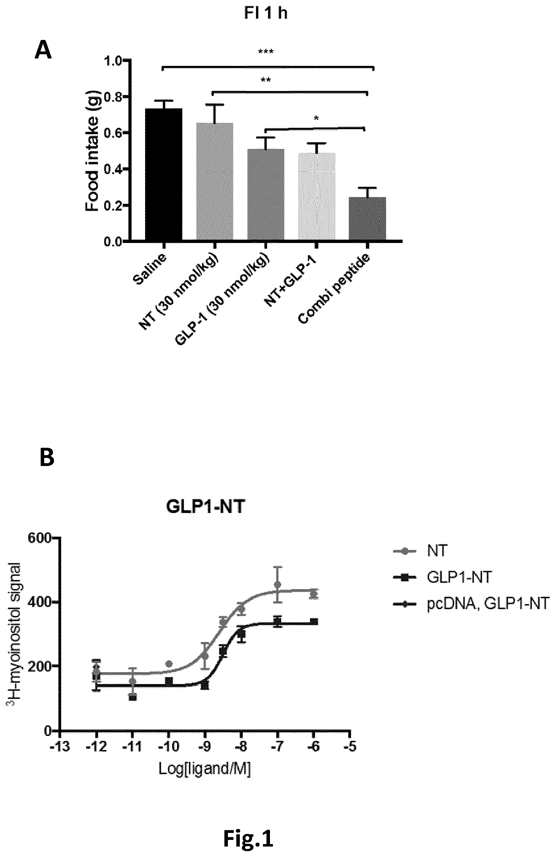

Thus, both NT and GLP-1 peptides are expressed in enteroendocrine cells of the small intestine and have an effect on appetite and food intake. However, it is known that these two peptides are most often not expressed simultaneously in the same cells. In the rare cases where they are, they are not processed in the same granulae. The inventors have shown a surprising synergistic decrease in food intake obtained with the fusion peptide of the invention, and this is significantly greater in comparison to the effect obtained with the co-administration of GLP-1 and NT, see Fig. 1. Thus, the administration of the dual agonist fusion peptide of the invention results in an unexpected improved negative regulation of appetite, food intake, and body weight.

In a preferred embodiment the fusion peptide comprises a first peptide comprising GLP-1 or an analogue thereof. GLP-1 (7-36) is shown in SEQ ID NO: 2. GLP-1 (7-37) is shown in SEQ ID NO: 44. GLP-1 analogues are shown in SEQ ID NO: 1 and SEQ ID NO: 3-31.

In another embodiment the fusion peptide comprises a first peptide comprising GLP-2 or an analogue thereof. GLP-2 is shown in SEQ ID NO: 35.

In another embodiment the fusion peptide comprises a first peptide comprising glucagon or an analogue thereof. Glucagon is shown in SEQ ID NO: 36. Glucagon has been shown to increase energy expenditure which contribute to body-weight loss.

In another embodiment the fusion peptide comprises a first peptide comprising vasoactive intestinal peptide (VIP) or an analogue thereof. VIP is shown in SEQ ID NO: 37.

In another embodiment the fusion peptide comprises a first peptide comprising secretin or an analogue thereof. Secretin is shown in SEQ ID NO: 38.

In another embodiment the fusion peptide comprises a first peptide comprising the pituitary adenylate cyclase-activating peptide 38 (PACAP-38) or an analogue thereof. PACAP-38 is shown in SEQ ID NO: 39. PACAP-38 has been shown to decrease the food intake by a central mechanism acting in the bed nucleus of the stria terminalis (BNST).

In another embodiment the fusion peptide comprises a first peptide comprising the Neurokinin-A peptide (NKA). NKA is shown in SEQ ID NO: 40. NKA or other ligands that activate the NK2 receptor may increase energy expenditure.

In another embodiment the fusion peptide comprises a first peptide comprising Peptide YY (PYY). PYY is shown in SEQ ID NO: 41. PYY may also decrease appetite by activation of the hypothalamic neurons.

In another embodiment the fusion peptide comprises a first peptide comprising th islet amyloid polypeptide (IAPP; amylin). Amylin is shown in SEQ ID NO: 42. Amylin may contribute to decreasing food intake through both central and peripheral mechanisms and indirectly by slowing gastric emptying.

The second peptide comprised within the fusion peptide of the invention and having at least 70% identity with any one of SEQ ID NO:24 to SEQ ID NO:31 may have at least about 75%, at least about 80%, at least about 85%, at least about 90%, at least about 95%, or more than about 97% identity to any one of SEQ ID NO:24 to SEQ ID NO:31. In particular, in a preferred embodiment, the second peptide is the peptide with the sequence laid out in SEQ ID NO:24.

In a preferred embodiment, the N-terminus of the fusion peptide is the first peptide of the fusion peptide. Thus, the N-terminus of the fusion peptide may have the amino acid sequence:

X₁-X₂-X₃-X₄-X₅-X₆-X₇-X₈-X₉

wherein
X₁ is L-histidine, D-histidine, desamino-histidine, 2-amino-histidine, β hydroxy-histidine, homohistidine, N^{α}-acetyl-histidine, α-fluoromethyl-histidine, α-methyl-histidine, 3-pyridylalanine, 2-pyridylalanine or 4 pyridylalanine;
X₂ is A, G, V, L, I, K, S, Aib, (1-aminocyclopropyl) carboxylic acid, (1 aminocyclobutyl) carboxylic acid, (1-aminocyclopentyl) carboxylic acid, (1 aminocyclohexyl) carboxylic acid, (1-aminocycloheptyl) carboxylic acid, or (1-aminocyclooctyl) carboxylic acid;
X₃ is E, D or Q;
X₄ is G or A;
X₅ is T, V, S or I;
X₆ is F or Y;
X₇ is T, or S;
X₈ is S, V, or D; and
X₉ is S, D, E, N or is not present.

In another preferred embodiment the amino terminus of the fusion peptide has the amino acid sequence laid out in SEQ ID NO:1, i.e. the amino terminus is the first peptide, which has the sequence of SEQ ID NO: 1.

Thus, in preferred embodiments the first peptide is forming the sequence of amino acids starting at the amino terminus of the fusion peptide of the invention, such that the amino acid in the most amino terminal position of the fusion peptide corresponds to the amino acid in the most amino terminal position of the first peptide. Analogously, the amino acid in the second position starting from the most amino terminal position of the fusion peptide corresponds to the amino acid in the second most amino terminal position of the first peptide. This correspondence is to be extended in an analogous manner along the length of the X₁-X₂-X₃-X₄-X₅-X₆-X₇-X₈-X₉ polypeptide or along the length of the polypeptide with the sequence laid out in SEQ ID NO:1.

In another preferred embodiment, the second peptide is the carboxyl terminus of the fusion peptide. Thus, the carboxyl terminus may have an amino acid sequence with at least 70% identity with any one of SEQ ID NO:24 to SEQ ID NO:31.

In another preferred embodiment, the carboxyl terminus is selected from the list consisting of:
X₁₀-X₁₁-P-X₁₂-I-L;
P-X₁₀-X₁₁-P-X₁₂-I-L;
K-P-X₁₀-X₁₁-P-X₁₂-I-L;
N-K-P-X₁₀-X₁₁-P-X₁₂-I-L;
E-N-K-P-X₁₀-X₁₁-P-X₁₂-I-L;
Y-E-N-K-P-X₁₀-X₁₁-P-X₁₂-I-L;
L-Y-E-N-K-P-X₁₀-X₁₁-P-X₁₂-I-L;
Q-L-Y-E-N-K-P-X₁₀-X₁₁-P-X₁₂-I-L; or
E-L-Y-E-N-K-P-X₁₀-X₁₁-P-X₁₂-I-L
wherein
X₁₀ is R or K;
X₁₁ is R or K; and
X₁₂ is Y, S, C or T.

In another preferred embodiment the carboxyl terminus of the fusion peptide has the amino acid sequence laid out in SEQ ID NO:24. Thus, in preferred embodiments the second peptide is forming the sequence of amino acids starting at the carboxyl terminus of the fusion peptide of the invention, such that the amino acid in the most carboxyl terminal position of the fusion peptide corresponds to the amino acid in the most carboxyl terminal position of the second peptide. Analogously, the amino acid in the second position starting from the most carboxyl terminal position of the fusion peptide corresponds to the amino acid in the second most carboxyl terminal position of the second peptide. This correspondence is to be extended in an analogous manner along the length of the polypeptide with the sequence laid out in any one of SEQ ID NO:24 to SEQ ID NO:31 or along the length of peptides X₁₀-X₁₁-P-X₁₂-I-L; P-X₁₀-X₁₁-P-X₁₂-I-L; K-P-X₁₀-X₁₁-P-X₁₂-I-L; N-K-P-X₁₀-X₁₁-P-X₁₂-I-L; E-N-K-P-X₁₀-X₁₁-P-X₁₂-I-L; Y-E-N-K-P-X₁₀-X₁₁-P-X₁₂-I-L; L-V-E-N-K-P-X₁₀-X₁₁-P-X₁₂-I-L; or Q-L-V-E-N-K-P-X₁₀-X₁₁-P-X₁₂-I-L wherein X₁₀ is R or K; X₁₁ is R or K; and X₁₂ is Y, S, C or T, in a carboxyl-terminus to amino-terminus direction. In the context of identity, the term "corresponds" is not to be interpreted as "is the same as". In this manner, if e.g. an amino acid is said to correspond to another, it is not meant that it is the same amino acid that is found at the same position in both peptides, but merely that the two amino acids in question are located at the same positions in the two peptides relative to the most amino terminal or carboxyl terminal residue. Thus, two amino acids at the same position, e.g. the third most carboxyl terminal position (e.g. X₁₂), may be a different amino acid in each of the fusion peptide of the invention and e.g. the peptide X₁₀-X₁₁-P-X₁₂-I-L.

It is particularly preferred that the N-terminus is the first peptide and that the C-terminus is the second peptide. When both the first and the second peptide are thus located at the respective termini, both of the functionalities provided by the first peptide and the second peptide are most readily available, and the synergistic effect will be optimal. It is further preferred that when a peptide is present as a linker molecule, e.g. a peptide having 4 to 10 amino acids, the N-terminus is the first peptide and that the C-terminus is the second peptide.

When the N-terminus is the first peptide and the C-terminus is the second peptide, the fusion peptide may have a total length of 15 amino acids, e.g. corresponding to the 9 amino acids of SEQ ID NO: 1 or a peptide with the sequence X₁-X₂-X₃-X₄-X₅-X₆-X₇-X₈-X₉ with X₁ to X₉ as defined above, linked directly to the 6 amino acids sequence of SEQ ID NO: 31 or a peptide with the sequence X₁₀-X₁₁-P-X₁₂-I-L with X₁₀ to X₁₂ as defined above. In another embodiment, the fusion peptide has a total length of 43 amino acids, e.g. corresponding to the 30 amino acids of SEQ ID NO: 2 linked directly to the 13 amino acids sequence of SEQ ID NO: 24 resulting in the 43 amino acid GLP-1 (7-36) NT (1-13) sequence laid out in SEQ ID NO: 32. The respective 15 amino acid fusion peptide and 43 amino acid fusion peptide may both contain in addition a linker molecule, e.g. a peptide having 4 to 10 amino acids.

In a preferred embodiment the fusion peptide of the invention has at least 70% identity with the fusion peptide with the sequence laid out in SEQ ID NO: 32. SEQ ID NO: 32 corresponds to SEQ ID NO: 2 directly linked to SEQ ID NO: 24.

The fusion peptide of the invention may, however, be a combination of any of SEQ ID NO: 1 to SEQ ID NO: 23 with any one of SEQ ID NO: 24 to SEQ ID NO: 31, such as, for example, SEQ ID NO: 43 or SEQ ID NO: 45.

In another preferred embodiment the fusion peptide of the invention has at least 70% identity with the fusion peptide with the sequence laid out in SEQ ID NO: 43. SEQ ID NO: 43 corresponds to GLP-1 (7-37) as laid out in SEQ ID NO: 44 directly linked to NT (1-13) as laid out in SEQ ID NO: 24.

Such fusion peptides may have at least about 75%, at least about 80%, at least about 85%, at least about 90%, at least about 95%, or more than about 97% identity to SEQ ID NO: 32 or SEQ ID NO: 43, respectively.

The fusion peptide of the invention may also be a combination of any of Exendin 4, GLP-2, Glucagon, VIP, Secretin, PACAP-38, NKA, PYY or Amylin, as laid out in SEQ ID NO: 34 to SEQ ID NO: 42, respectively, or GLP-1 (7-37) as laid out in SEQ ID NO: 44 with any of NT (1-13) to NT (8-13) as laid out in SEQ ID NO: 24 to SEQ ID NO: 31, respectively, forming the fusion peptide of the invention such as, for example, that laid out in SEQ ID NO: 33, SEQ ID NO: 43 or SEQ ID NO: 46.

In another preferred embodiment the fusion peptide of the invention has at least 70% identity with an Exendin-4/NT fusion peptide or the peptide with the sequence laid out in SEQ ID NO: 33. In such a case, when the N-terminus is the first peptide and the C-terminus is the second peptide, the fusion peptide may have a total length of 45 amino acids, e.g. corresponding to the 39 amino acids of SEQ ID NO: 34 linked directly to the 6 amino acids sequence of SEQ ID NO: 31 or a peptide with the sequence X₁₀-X₁₁-P-X₁₂-I-L with X₁₀ to X₁₂ as defined above. In another embodiment, the fusion peptide has a total length of 52 amino acids, e.g. corresponding to the 39 amino acids of SEQ ID NO: 34 linked directly to the 13 amino acids sequence of SEQ ID NO: 24 or a peptide with the sequence Q-L-Y-E-N-K-P-X₁₀-X₁₁-P-X₁₂-I-L with X₁₀ to X₁₂ as defined above. The respective 45 amino acid fusion peptide and 53 amino acid fusion peptide may both contain a linker molecule, e.g. a peptide having 4 to 10 amino acids. Such fusion peptides may have at least about 75%, at least about 80%, at least about 85%, at least about 90%, at least about 95%, or more than about 97% identity to SEQ ID NO: 33. In particular, in a preferred embodiment, the fusion peptide is the polypeptide with the sequence laid out in SEQ ID NO: 33. Exendin-4/NT polypeptides, as examples of the fusion peptide of the invention, have an extended plasma half-life in comparison to that of GLP-1 or NT peptides.

In another preferred embodiment the fusion peptide of the invention has at least 70% identity with a GLP-1 (7-36) / NT (2-13) polypeptide or the peptide with the sequence laid out in SEQ ID NO: 45. In such a case, when the N-terminus is the first peptide and the C-terminus is the second peptide, the fusion peptide may have a total length of 42 amino acids, e.g. corresponding to the 30 amino acids of SEQ ID NO: 2 linked directly to the 12 amino acids sequence of SEQ ID NO: 25 or a peptide with the sequence L-Y-E-N-K-P-X₁₀-X₁₁-P-X₁₂-I-L with X₁₀ to X₁₂ as defined above. The 42 amino acid fusion peptide may contain in addition a linker molecule, e.g. a peptide having 4 to 10 amino acids. Such fusion peptides may have at least about 75%, at least about 80%, at least about 85%, at least about 90%, at least about 95%, or more than about 97% identity to SEQ ID NO: 45. In particular, in a preferred embodiment, the fusion peptide is the polypeptide with the sequence laid out in SEQ ID NO: 45. GLP-1 (7-36) / NT (2-13) polypeptides, as examples of the fusion peptide of the invention, have an extended plasma half-life in comparison to that of GLP-1 or NT peptides.

In preferred embodiment, the fusion peptide comprises a first peptide which is H-A-E-G-T-F-T-S-D-V-S-S-Y and a second peptide which is E-L-Y-EN-K-P-R-R-P-Y-I-L.

In preferred embodiment, the fusion peptide comprises a first peptide which is H-A-E-G-T-F-T-S-D-V-S-S-Y-L-E-G-Q and a second peptide which is E-L-Y-E-N-K-P-R-R-P-Y-I-L.

In preferred embodiment, the fusion peptide comprises a first peptide which is H-A-E-G-T-F-T-S-D-V-S-S-Y-L-E-G-Q-A-A-K and a second peptide which is E-L-V-E-N-K-P-R-R-P-V-I-L.

In preferred embodiment, the fusion peptide comprises a first peptide which is H-A-E-G-T-F-T-S-D-V-S-S-Y-L-E-G-Q-A-A-K-E-F-I-A-W-L-V-K-G-R and a second peptide which is E-L-V-E-N-K-P-R-R-P-V-I-L.

In preferred embodiment, the fusion peptide comprises a first peptide which comprises H-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises D-histidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises desamino-histidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises 2-amino-histidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises β-hydroxy-histidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises homohistidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises N°-acetyl-histidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises α-fluoromethyl-histidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises α-methyl-histidine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises 3-pyridylalanine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises 2-pyridylalanine-1-aminocyclopropyl-E-G-T-F-T-S-D-V and a second peptide which is E-L-V-E-N-K-P-R-R-P-V-I-L.

In preferred embodiment, the fusion peptide comprises a first peptide which comprises 4-pyridylalanine-1-aminocyclopropyl-E-G-T-F-T-S-D-V and a second peptide which is E-L-Y-E-N-K-P-R-R-P-Y-I-L.

In a preferred embodiment, the fusion peptide is the polypeptide with the sequence laid out in SEQ ID NO: 32.

In another preferred embodiment, the fusion peptide is the polypeptide with the sequence laid out in SEQ ID NO: 43.

In another preferred embodiment, the fusion peptide is the polypeptide with the sequence laid out in SEQ ID NO: 45.

In another preferred embodiment, the fusion peptide is the polypeptide with the sequence laid out in SEQ ID NO: 46.

Percent identity between two peptides found throughout this document may be determined using any of the methods described herein that align the polypeptides or fragments being compared, and determine the extent of amino acid identity or similarity between them, such as preferably the BLAST method.

Amino acid or nucleotide sequences of the present invention that are identical to other polypeptide or nucleotide sequences to a certain percentage must comprise enough of the amino acid sequence of a polypeptide or the nucleotide sequence of a gene to afford putative identification of that polypeptide or gene, either by manual evaluation of the sequence by one skilled in the art, or by computer-automated sequence comparison and identification using algorithms such as BLAST (Basic Local Alignment Search Tool) (for a review see Altschul, et al., Meth Enzymol. 266: 460,1996; and Altschul, et al., Nature Genet. 6: 119, 1994).

BLAST is the heuristic search algorithm employed by the programs BLASTP, BLASTN, BLASTX,TBLASTN, and TBLASTX using the statistical methods of Karlin and Altschul (available at www.ncbi.nih.gov/BLAST) Altschul, et al., J. Mol. Biol. 215: 403,1990). The BLAST programs were tailored for sequence similarity searching, for example to identify homologues to a query sequence.

For the present invention the BLAST program is applied under standard algorithms; General parameters; "Short queries" - on/mark, "Expect threshold" - 10, "word size" - 3, and "max matches in a query range" - 0. Scoring parameters; "Matrix" - BLOSUM62, and "Gap costs" - Existence 11 Extension 1. Filter and masking; off/no mark in "low complexity regions", off/no mark in "mask for lookup table only", and off/no mark in "mask lower case letters".

For use in alignment purposes the amino (N-) and carboxyl (C-) termini are to be used. These positions can be identified in all sequences. Other computer program methods to determine identity and similarity between the two sequences include but are not limited to the GCG program package (Devereux, et al., Nucl. Acids Res. 12: 387,1984) and FASTA (Atschul, et al., J Molec. Biol. 215: 403,1990).

By "percentage identity" is meant % of identical amino acids between the two compared proteins. By "% similarity" is meant the percentage of similar amino acids between the two compared proteins.

One skilled in the art can purify a fusion peptide using standard techniques for protein purification to obtain a fusion peptide that is substantially pure.

As used herein, the term "substantially pure" refers to fusion peptides which are substantially free of other proteins, lipids, carbohydrates or other materials with which it is naturally associated. The fusion peptides can be analysed by standard SDS-PAGE and/or immunoprecipitation analysis and/or Western blot analysis, for example. The purity of a fusion peptide can also be determined by amino terminal amino acid sequence analysis.

In yet another embodiment the first peptide is selected from SEQ ID NO: 3, which correspond to GLP-1 (7-35), SEQ ID NO: 2, which correspond to GLP-1 (7-36), GLP-1 (7-37), GLP-1 (7-38), GLP-1 (7-39), GLP-1 (7-40), GLP-1 (7-41), respectively, or an analogue thereof.

In another embodiment the first peptide is a fragment of a peptide selected from the group comprising SEQ ID NO: 3, which corresponds to GLP-1 (7-35), SEQ ID NO: 2, which corresponds to GLP-1 (7-36), GLP-1 (7-37), GLP-1 (7-38), GLP-1(7-39), GLP-1(7-40) and GLP-1(7-41) or an analogue thereof.

In another embodiment the first peptide comprises no more than fifteen amino acid residues which have been exchanged, added or deleted as compared to SEQ ID NO: 2 (GLP-1 (7-36)), or no more than ten amino acid residues which have been exchanged, added or deleted as compared to SEQ ID NO: 2 (GLP-1 (7-36)).

In another embodiment the first peptide is SEQ ID NO: 34 (Exendin-4).

In one embodiment, the fusion peptide comprises a linker molecule, e.g. the first peptide may be linked to the second peptide by a linker molecule. Thus, the first peptide may be linked directly to the second peptide, or there may be a linker between the first peptide and the second peptide. The linker molecule may have any chemistry as desired. For example, the linker molecule may be a peptide, e.g. of 4 to 10 amino acids. When the linker molecule is a peptide, it is preferred that the amino acids neutral or basic amino acids or a combination of neutral or basic amino acids. In a preferred embodiment the linker molecule is a peptide comprising the sequence K-K-G-G. By including a linker, steric hindrance at the binding sites of the peptide may be reduced and overall increased potency of the fusion peptide may be achieved.

In one embodiment the C-terminal end of the fusion peptide is amidated. In comparison with C-terminal acids, C-terminal amides may considerably enhance peptide activity. Without wishing to be bound by any particular theory, it is believed that amidation of the C-terminus may increase the basicity of the carboxyl terminal carboxylic acid. Overall solubility of the fusion peptide may not be improved in comparison to that of C-terminal acids, but the presence of a C=O dipole may allow the amide to act as a H-bond acceptor from water. The presence of N-H dipoles also allows amides to function as H-bond donors. Thus, amides can participate in hydrogen bonding with water and other protic solvents, which may be advantageous for the interaction between the carboxyl terminus of the fusion peptide and the NT receptor. Furthermore, this modification may increase the metabolic stability of the fusion peptides of the invention as well as their ability to resist enzymatic degradation by, e.g. aminopetidases, exopeptidases, and synthetases.

In yet another embodiment the second peptide is selected from SEQ ID NO: 24 to SEQ ID NO: 31, which correspond to NT (1-13), NT (2-13), NT (3-13), NT (4-13), NT (5-13), NT (6-13), NT (7-13), NT (8-13), respectively, or an analogue thereof.

In another embodiment the second peptide is a fragment of SEQ ID NO: 24, i.e. the NT (1-13) peptide, or an analogue thereof.

In another embodiment the second peptide comprises an albumin binding moiety attached via a spacer to an amino acid residue.

In another embodiment the second peptide comprises no more than six amino acid residues which have been exchanged, added or deleted as compared to SEQ ID NO: 24 (NT (1-13), or no more than four amino acid residues which have been exchanged, added or deleted as compared to SEQ ID NO:24 (NT (1-13), and optionally one albumin binding moiety attached via a spacer to an amino acid residue.

In another aspect of the invention, the first peptide has been linked to an albumin binding moiety via a spacer. The linking may be covalent or non-covalent. Non-covalent association of a peptide with albumin may extend the half-life of short lived proteins.

In one embodiment, the albumin binding moiety is a lipophilic moiety. In a further embodiment, the lipophilic moiety is attached, e.g. covalently attached, to a lysine residue optionally via a linker by conjugation chemistry such as by alkylation, acylation, ester formation, or amide formation or to a cysteine residue by thiol maleimide coupling.

In another embodiment, the albumin binding moiety is negatively charged at physiological pH. In another embodiment, the albumin binding moiety comprises a group which can be negatively charged. One such preferred group which can be negatively charged is a carboxylic acid group.

In another embodiment, the albumin binding residue binds non-covalently to albumin.

In another embodiment the albumin binding residue has a binding affinity (K_{d}) towards human serum albumin that is below about 10 µM or below about 1 µM. The binding the albumin binding residue to albumin is a reversible process, and the rate of the binding reaction is proportional to the concentrations of the reactants. K_{d} is the ratio of the albumin binding residue dissociation rate (k_{off}), how quickly it dissociates from albumin, to the association rate (kₒₙ) of albumin binding residue, how quickly it binds to albumin.

In yet another embodiment, the albumin binding moiety is selected from the group consisting of a straight chain alkyl group, a branched alkyl group, a group which has an ω-carboxylic acid group, and a partially or completely hydrogenated cyclopentanophenanthrene tetracyclic skeleton.

In a further embodiment, the albumin binding moiety is a cibacronyl residue.

In another embodiment, the albumin binding moiety has from 6 to 40 carbon atoms, from 8 to 26 carbon atoms or from 8 to 20 carbon atoms.

In another embodiment, the albumin binding moiety is an acyl group selected from the group comprising CH₃(CH₂)ᵣCO-, wherein r is an integer from 4 to 38, preferably an integer from 4 to 24, more preferred selected from the group comprising CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

In another embodiment of the invention, the albumin binding moiety is an acyl group of a straight-chain or branched alkane α,ω-dicarboxylic acid.

In another embodiment the albumin binding moiety is an acyl group selected from the group comprising HOOC(CH₂)_{S}CO-, wherein s is an integer from 4 to 38, preferably an integer from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂₂CO-.

In another embodiment the albumin binding moiety is a group of the formula CH₃(CH₂)ᵥCO-NHCH(COOH)(CH₂)₂CO-, wherein v is an integer of from 10 to 24.

In another embodiment the albumin binding moiety is a group of the formula CH₃(CH₂)_{w}CO-NHCH((CH₂)₂COOH)CO-, wherein w is an integer of from 8 to 24.

In another embodiment the albumin binding moiety is a group of the formula COOH(CH₂)ₓCO- wherein x is an integer of from 8 to 24.

In another embodiment the albumin binding residue is a group of the formula -NHCH(COOH)(CH₂)₄NH-CO(CH₂)_{y}CH-, wherein y is an integer of from 8 to 18.

In another embodiment of the invention the albumin binding moiety is a peptide, such as a peptide comprising less than 40 amino acid residues. A number of small peptides which are albumin binding moieties as well as a method for their identification is found in J. Biol Chem. 277, 38 (2002) 35035-35043.

In another embodiment the albumin binding residue linked via a spacer is attached to said fusion peptide via the ε-amino group of a lysine residue.

In another particular embodiment, the fusion peptide according to the invention comprises a spacer between the fusion peptide sequence and one or more albumin binding moiety(s). The spacer may be, e.g. one or more unbranched oligo ethylene glycol (OEG) moiety(s) with appropriate functional groups at both terminals, which form a bridge between an amino group of the fusion peptide sequence and a functional group of the albumin binding moiety. Another appropriate spacer may be amino-3,6-dioxaoctanoic acid (ADO) Appropriate functional groups on the end of the spacer linking the peptide include but are not limited to -C(O)NH-, -NHC(O)-, -C(O)NHCH₂, - CH₂NHC(O)-, -OC(O)NH-, -NHC(O)O-, -C(O)NHCH₂, CH₂NHC(O)-, - C(O)CH₂, -CH₂C(O)-, -C(O)CH=CH-, -CH=CHC(O)-, -(CH₂)₉-, -C(O)-, - C(O)O-, -OC(O)-, -NHC(O)- and -C(O)NH-.

In another embodiment the albumin binding moiety via spacer and linkers is attached to said fusion peptide via an amino acid residue. In one embodiment the albumin binding residue is attached to said fusion peptide via a cysteine residue.

In another embodiment the albumin binding residue via spacer and linkers is attached to said fusion peptide via an amino acid residue. In one embodiment the albumin binding residue is attached to said fusion peptide via a glutamate residue.

In another embodiment the albumin binding residue via spacer and linkers is attached to said fusion peptide via an amino acid residue. In one embodiment the albumin binding residue is attached to said fusion peptide via a gamma-Glutamylglutamic acid (γ-Glu) dipeptide.

In one embodiment the albumin binding residue is attached to said fusion peptide via an aspartate residue.

Combinations of spacers may also be advantageous, as it is known that different spacers between a peptide and an albumin binding moiety may affect the peptide-receptor binding affinity. Consequently, said spacer may comprise combinations of spacers such as 2x-OEG, γ-Glu-OEG, γ-Glu-2xOEG, γ-Glu-3xOEG, D-γ-Glu-OEG, DγGlu-2xOEG, 2xγGlu-2xOEG, 3xγGlu-2xOEG, Abu-γGlu-OEG, Abu-2xγGlu-OEG, Abu-2xOEG or Benzyl-βAla-2xOEG.

In another embodiment the fusion peptide of the invention is PEGylated (i.e. the fusion peptide has one or more poly-ethylene glycol, PEG, moieties). PEGylation involves covalent and non-covalent attachment or amalgamation of polyethylene glycol polymer chains to the fusion peptide. PEGylated fusion peptides of the invention may have prolonged blood circulation half-lives, improved drug solubility and stability, and reduced immunogenicity. Without wishing to bound to any particular theory, each ethylene glycol subunit in PEG is believed to associate with two to three water molecules making PEGylated molecules about five to ten times larger than a soluble protein of a similar molecular mass. Because the kidneys filter substances based on size, PEGylated molecules that have a higher molecular weight and larger hydrodynamic radius than the parent molecule are cleared from the body at a much slower rate. This decreased rate increases the half-life of the PEGylated molecule in vivo. In addition to having a fast clearance in vivo, many native type proteins and peptides are also rapidly degraded by circulating enzymes via proteolysis, including the fusion peptide of the invention. The hydrated PEG chain protects the conjugated compound from access to proteases and peptidases by steric hindrance and therefore reduces the drug's nonspecific degradation.

There are several strategies for coupling PEG to peptides (see, e.g. Veronese, Biomaterials 22:405-417, 2001). Those skilled in the art, will therefore be able to utilize well-known techniques for linking the PEG polymer to GLP-1 peptides described herein.

Briefly, cysteine PEGylation is one method for site-specific PEGylation, and can be accomplished by introducing a unique cysteine mutation at one of the specific positions in the native peptide sequences and then reacting the resulting peptide with a cysteine-specific PEGylation reagent, such as PEG-maleimide. Thus, it may be necessary to mutate the peptide in order to allow for site-specific PEGylation. For example, if the peptide contains cysteine residues, these will need to be substituted with conservative amino acids in order to ensure site-specific PEGylation. In addition, linker molecules may be added to the linker region joining first peptide and the second peptide (i.e. including a unique cysteine residue).

In a particular embodiment, the PEG polymer has a molecular weight greater than 700Da, in other embodiments a molecular weight greater than 5 kDa, greater than 10 kDa, or greater that 20kDa. The PEG polymer may be linear or branched. In cases where the PEG polymer is greater than 20kDa, the PEG polymer preferably has a branched structure, such as for example, a 43 kDa branched PEG molecule (Shearwater 2001 catalogue #2D3XOT01, mPEG2-MAL).

To reduce steric hindrance of the PEG derivatised fusion peptide of the invention, the attachment of a PEG can be effected on the opposite side of the peptide surface that interacts with GLP-1R and NT receptors.

In a second aspect, the present invention relates to a pharmaceutical composition comprising the fusion peptide according to the invention, and a pharmaceutically acceptable carrier.

In one embodiment, the pharmaceutical composition is suited for parenteral administration.

The fusion peptide or the pharmaceutical composition as described herein above are suitable for treating diseases of the liver, such as non-alcoholic fatty liver disease (NAFLD) or non-alcoholic steatohepatitis (NASH). NAFLD is characterized by fatty liver related to insulin resistance in the absence of significant alcohol consumption. It embraces a pathological spectrum from simple steatosis to steatohepatitis. Non-alcoholic steatohepatitis (NASH) can progress to cirrhosis and hepatocellular carcinoma. The administration of the fusion peptide or the pharmaceutical composition as described herein above may help alleviate NAFLD, cirrhosis and/or hepatocellular carcinoma in a mammalian patient.

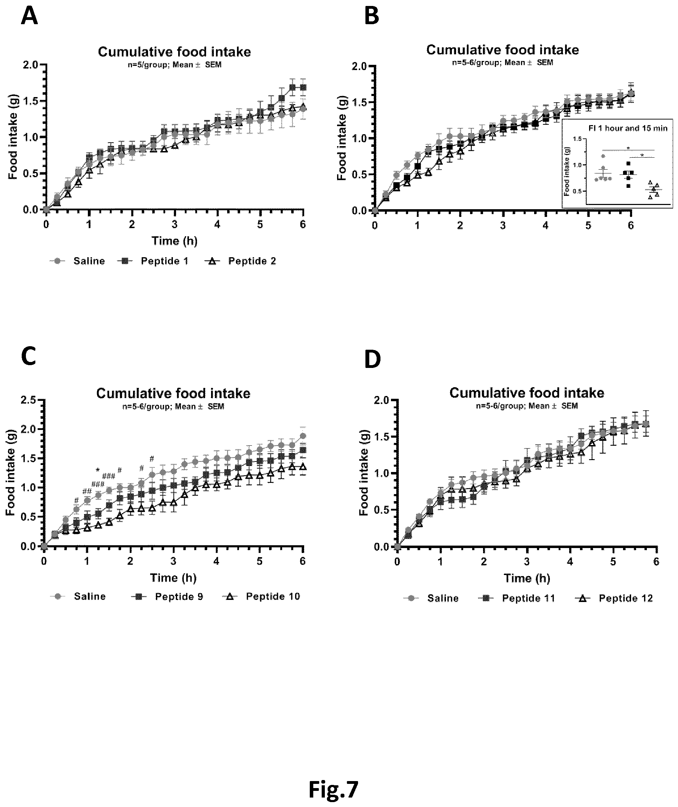

The fusion peptide or the pharmaceutical composition as described herein above are suitable for treating cardiovascular diseases, such as non-fatal myocardial infarction or ischaemic vascular disorders including stroke, or cardiovascular oedema. Proprotein convertase subtilisin/kexin type 9 (PCSK9) expression is decreased in the liver upon administration of the peptide of the invention (see Fig. 7). PCSK9 inhibitors has been shown to be highly efficient for hypercholesterolemia and prevent cardiovascular diseases. Thus, the administration of the fusion peptide or the pharmaceutical composition as described herein above may help alleviate cardiovascular diseases in a mammalian patient.

The present invention relates to a pharmaceutical composition comprising a fusion peptide according to the invention, and a pharmaceutically acceptable carrier. Whilst it is possible for the compounds or salts of the present invention to be administered as the raw peptide or peptide analogue, it is preferred to present them in the form of a pharmaceutical formulation. Accordingly, the present invention further provides a pharmaceutical formulation, which comprises a fusion peptide of the present invention or a pharmaceutically acceptable salt or ester thereof, and a pharmaceutically acceptable carrier therefor. The pharmaceutical formulations may be prepared by conventional techniques, e.g. as described in Remington: The Science and Practice of Pharmacy 2005, Lippincott, Williams & Wilkins. Briefly, pharmaceutically acceptable carriers can be either solid or liquid. Solid form preparations include powders, tablets, pills, capsules, cachets, suppositories, and dispersible granules. A solid carrier can be one or more excipients which may also act as diluents, flavouring agents, solubilisers, lubricants, suspending agents, binders, preservatives, wetting agents, tablet disintegrating agents, or an encapsulating material.

The active ingredient may be in powder form, obtained by aseptic isolation of sterile solid or by lyophilisation from solution for constitution before use with a suitable vehicle, e.g., sterile, pyrogen-free water.

In one embodiment, the pharmaceutical composition is suited for parenteral administration. Accordingly, the compositions of the present invention may be presented in unit dose form in ampoules, pre-filled syringes, small volume infusion or in multi-dose containers, optionally with an added preservative. The compositions may take such forms as suspensions, solutions, or emulsions in oily or aqueous vehicles, for example solutions in aqueous poly-ethylene glycol. Examples of oily or non-aqueous carriers, diluents, solvents or vehicles include propylene glycol, polyethylene glycol, vegetable oils (e.g., olive oil), and injectable organic esters (e.g., ethyl oleate), and may contain agents such as preserving, wetting, emulsifying or suspending, stabilizing and/or dispersing agents.

In another aspect the present invention relates the fusion peptide or the pharmaceutical composition as described herein above suitable for treating diseases. The treatment with a fusion peptide according to the present invention may also be combined with a second or further pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are: Insulin, sulphonylureas, biguanides, meglitinides, glucosidase inhibitors, glucagon antagonists, DPP-IV (dipeptidyl peptidase-IV) inhibitors, inhibitors of hepatic enzymes involved in stimulation of gluco-neogenesis and/or glycogenosis, glucose uptake modulators, compounds modifying the lipid metabolism such as antihyperlipidemic agents as HMG CoA inhibitors (statins), Gastric Inhibitory Polypeptides (GIP analogues), compounds lowering food intake, RXR agonists and agents acting on the ATP-dependent potassium channel of the [beta]-cells; Cholestyramine, coles-tipol, clofibrate, gemfibrozil, lovastatin, pravastatin, simvastatin, probucol, dextrothyroxine, neteglinide, repaglinide; [beta]-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and vera-pamil, and [alpha]-blockers such as doxazosin, urapidil, prazosin and terazosin; CART (cocaine amphetamine regulated transcript) agonists, NPY (neuropeptide Y) antagonists, PYY agonist, PYY2 agonists, PYY4 agonists, mixed PPY2/PYY4 agonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, [beta]3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR [beta] agonists; histamine H3 antagonists, Gastric Inhibitory Polypeptide agonists or antagonists (GIP analogs), gastrin and gastrin analogs. The treatment with a compound according to this invention may also be combined with surgery- a surgery that influence the glucose levels and/or lipid homeostasis such as gastric banding or gastric bypass.

It should be understood that any suitable combination of the compounds according to the invention with one or more of the above-mentioned compounds and optionally one or more further pharmacologically active substances are considered to be within the scope of the present invention.

Neurotensin and GLP-1 act on their respective receptor with similar affinity in the nanomolar range. Suitable doses range from at least about 3.5 ng of active fusion peptide (i.e., not including the weight of a PEG or albumin binding moiety)/kg body weight to about 300 mg/kg body weight per day. In most cases, the dosage is from about 0.1 µg/kg to about 35 µg/kg (i.e., 0.1, 1, 5, 10, 15, 20, 25, 30, and 35 µg/kg) body weight daily, taking into account the routes of administration, symptoms, etc. Determination of a dose is well within the skill of the ordinary artisan and requires only routine screening.

Data described in the present invention can be translated to humans, since the hormone system is similar in these two species, which display the same receptor expression. Further, the regulation of GLP-1 and NT is regulated in a similar manner in both humans and mice. Increased expression is observed after a meal and strong increase in secretions is also observed in relation to gastric bypass surgery.

In another aspect the invention concerns a polynucleotide encoding a polypeptide as defined herein.

In another aspect the invention concerns a vector comprising a polynucleotide encoding a polypeptide as described herein.

In another aspect the invention concerns a host cell, such as a bacterial host cell, a mammalian host cell, such as a human host cell, comprising a polynucleotide or a vector as described herein.

In an embodiment the invention concerns a composition, preferably a pharmaceutically acceptable composition, comprising any one or more of a fusion peptide or fusion peptide derivative, a polynucleotide, a vector, or a host cell as described herein.

Described herein is a polypeptide or polypeptide derivative as described herein, a composition, a polynucleotide, a vector, or a host cell as described herein for use in the manufacture of a medicament.

Described herein is a method for treatment or prophylaxis of diseases of the liver, such as non-alcoholic fatty liver disease (NAFLD) or non-alcoholic steatohepatitis (NASH), or a method for treatment or prophylaxis of cardiovascular diseases, such as non-fatal myocardial infarction or ischaemic vascular disorders including stroke, or cardiovascular oedema, said method comprising the administration of a fusion peptide or fusion peptide derivative as described herein, a composition, a polynucleotide, a vector, or a host cell as described herein to a subject in need thereof.

Described herein is a method for regulating food intake, body weight, energy expenditure and/or appetite, said method comprising the administration of a fusion peptide or fusion peptide derivative as described herein, a composition, a polynucleotide, a vector, or a host cell as described herein to a subject in need thereof.

### Brief Description of Drawings

Fig. 1 shows the effect of GLP-1, NT and GLP-1/NT combi peptide on food intake.
Fig. 2 shows the effect of NT alone and NT+NT antagonist on food intake.

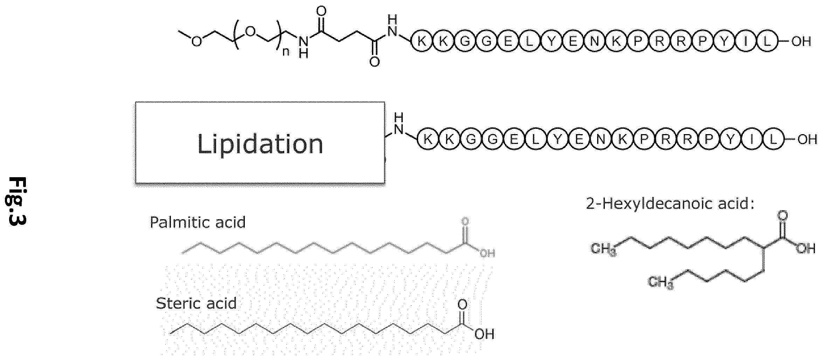

Fig. 3 represents PEGylated and lipidated NT peptides.
Fig. 4 shows the effect of NT and Liraglutide on body weight, food intake and body composition.
Fig. 5 shows in-vitro potency and efficacy of PEG-NT on inositol phosphate accumulation and food intake.
Fig. 6 shows the effect of NT and Liraglutide on selected gene expression levels in the liver.
Fig 7. shows how doses of 100 nmol/kg of the shortened GLP-1-NT combination peptides effects food intake in lean mice.
Fig 8. shows that a full Peg-NT-GLP-1 combination peptides at different dose levels reduces acute food intake and body weight in diet induced obesity (DIO) mice.
Fig 9. shows that a full Peg-NT-GLP-1 (100 nmol/kg) combination peptides reduces cumulative food intake and induces body weight loss relative to both saline and Peg-NT (100 nmol/kg) after sub-chronic treatment in DIO mice.

### Description of Embodiments

The term "polypeptide" and "peptide" as used herein means a compound composed of at least six constituent amino acids connected by peptide bonds. The constituent amino acids may be from the group of the amino acids encoded by the genetic code and they may be natural amino acids which are not encoded by the genetic code, as well as synthetic amino acids.

A peptide or polypeptide will have an amino terminus and a carboxyl terminus. In the context of the invention, the amino terminus and a carboxyl terminus may also be referred to as the N-terminus and the C-terminus, respectively, and corresponding derived forms.

Natural amino acids, which are not encoded by the genetic code, comprise e.g. hydroxyproline, γ-carboxyglutamate, ornithine, phosphoserine, D-alanine and D-glutamine. Synthetic amino acids comprise amino acids manufactured by chemical synthesis, e.g. D-isomers of the amino acids encoded by the genetic code such as D-alanine and D-leucine, a-aminoisobutyric acid (Aib), a-aminobutyric acid (Abu), tert-butylglycine (Tie), p-aianine, 3-aminomethyl benzoic acid, and anthranilic acid.

Peptide synthesis may be carried out by methods that are well known to the person skilled in the art. Briefly, peptides may be synthesized on Fmoc protected Rink amide resin or a similar resin suitable for solid phase peptide synthesis. Boc chemistry may be used; alternatively, protection amines can also be accomplished in acetonitrile solution using 4-dimethylaminopyridine (DMAP) as base. The Fmoc strategy using the FastMoc UV protocols employing HBTU (2-(1H-Benzotriazol-1-yl)-1,1,3,3 tetramethyluronium hexafluorophosphate) mediated couplings in N-methyl pyrrolidone and UV monitoring of the deprotection of the Fmoc protection group is yet another appropriate method.

Other coupling reagents besides from HBTU and HATU as described in e.g. Current Opinion in Chemical Biology, 2004, 8:211-221 may also be used.

The attachment of sidechains and linkers to specific lysine residues on the crude resin bound protected peptide may eventually be introduced in a specific position by incorporation of Fmoc-Lys(Dde)-OH during automated synthesis followed by selective deprotection with hydrazine. Other orthogonal protecting groups may be used on Lysine.

The first peptide of the fusion peptide of the invention may also be referred to using the term "GLP-1 peptide", and its derived forms, e.g. GLP-1 [7-36] (i.e. SEQ ID NO:2), GLP-1 analogue, GLP-1 derivative or a derivative of a GLP-1 analogue. GLP-1 analogues or derivatives of GLP-1 analogues may also be considered to be appetite regulating hormone peptides, e.g. Exendin-4, GLP-2, Glucagon, VIP, Secretin or PACAP-38 amongst others, or NKA, amylin, or PYY, which would also be suitable.

In one embodiment the first peptide is an insulinotropic agent.

The second peptide of the fusion peptide of the invention may also be referred to using the term "NT peptide", and its derived forms, e.g. neurotensin (NT) [1-13] (i.e. SEQ ID NO:24), NT analogue, NT derivative or derivative of a NT analogue.

The term "analogue" as used herein referring to a polypeptide means a modified peptide wherein one or more amino acid residues of the peptide have been substituted by other amino acid residues and/or wherein one or more amino acid residues have been deleted from the peptide and/or wherein one or more amino acid residues have been added to the peptide. Such addition or deletion of amino acid residues can take place at the N-terminal of the peptide and/or at the C-terminal of the peptide. Formulae of peptide analogues and derivatives thereof are drawn using standard single letter abbreviation for amino acids used according to IUPAC-IUB nomenclature.

The term "derivative" as used herein in relation to a peptide means a chemically modified peptide or an analogue thereof, wherein at least one substituent is not present in the unmodified peptide or an analogue thereof, i.e. a peptide which has been covalently modified. Typical modifications are amides, carbohydrates, alkyl groups, acyl groups, esters and the like.

The term "albumin binding moiety" as used herein means a moiety which binds non-covalently to human and/or other animal serum albumin. The albumin binding moiety attached to the fusion peptide typically has an affinity (as defined herein above) below 10 µM to human serum albumin and preferably below 1 pM. A range of albumin binding moieties are known among linear and branched lipophilic moieties containing 4-40 carbon atoms, compounds with a cyclopetanophenanthrene skeleton, peptides having 10-30 amino acid residues etc.

The term "spacer" as used herein means a spacer that separates a peptide and an albumin binding moiety with a chemical moiety which comprises at least 5 non-hydrogen atoms where 30-50% of these are either N or O.

The term "therapeutic polypeptide" as used herein means a fusion peptide which is being developed for therapeutic use, or which has been developed for therapeutic use.

The term "insulinotropic agent" as used herein means a compound which is an agonist of the human GLP-1 receptor, i.e. a compound which stimulates the formation of cAMP in a suitable medium containing the human GLP-1 receptor. The potency of an insulinotropic agent is determined by calculating the EC50 value from the dose-response curve.

### Examples

### Example 1: GLP-1/NT fusion peptide reduces food intake synergisticly.

As shown in Fig.1, both condition 5) (GLP-1/Neurotensin fusion peptide) and condition 4) (Neurotensin + GLP-1) show a lower food intake when compared to condition 2) (Neurotensin) and 3) (GLP-1), where each of the peptides are administrated separately, indicating a synergistic effect of the co-administration. Importantly, condition 5) (Neurotensin/GLP-1 fusion peptide) show a greater effect in lowing the food intake compared to condition 4) (Neurotensin + GLP-1) indicating an improved effect by the fusion peptide, where the peptide is constrained and may activate the two individual receptors (the neurotensin receptor and the GLP-1 receptor) simultaneously.

Lean eight weeks old C57BI/6J mice (Janvier) were single housed in metabolic cages (TSE systems) in a temperature controlled room with a 12:12h light dark cycle (lights on at 6:00) with ad libitum access to tap water and a chow diet (#1310, Altromin). Mice were acclimatized to the cages before peptides were tested.

For peptide testing, mice were randomized into the following groups according to their food intake during acclimatization:
1) Vehicle (saline) n=6
2) Neurotensin (30 nmol/kg) n=6
3) GLP-1 (30 nmol/kg) n=7
4) Neurotensin (30 nmol/kg) + GLP-1 (30 nmol/kg) n=6
5) Neurotensin/GLP-1 fusion peptide (30 nmol/kg) n=7

Peptides were dosed subcutaneously in a volume of 10 ml/kg in the early light phase after overnight fasting in a cross-over design. Food intake was monitored automatically after dosing and the mice were allowed a washout period of 5 days between dosings. As shown in Fig.1, condition 5) (Neurotensin/GLP-1 fusion peptide) shows a lower food intake when compared to condition 4) (Neurotensin + GLP-1) indicating a synergistic effect of the fusion peptide despite a 1:2 molar ratio of administered peptide in the two conditions.

### Example 2: The GLP-1/NT fusion peptide is a potent NTSR1 agonist.

The neurotensin receptor (NTS-R1) is described as a primarily Gα_{q/11}-coupled receptor.

Human Embryonic Kidney (HEK) 293 cells were cultured at 10 % CO₂, 90 % humidity and 37°C in Dulbecco's Modified Eagle Medium with GlutaMAX (Gibco) supplemented with 10 % fetal bovine serum (FBS), 100 U/ml penicillin and 0.1 mg/ml streptomycin.

For inositol phosphate (IP) accumulation assay, HEK293 cells were seeded in poly-D-lysine-coated 96-well-plates (PerkinElmer) the day before transfections, at a density of 30,000 cells per well. Transient transfections were performed using Lipofectamine 2000 (Thermo Fisher) according to the manufacturer's instructions. Cells were transfected with 20 ng DNA (pCMV-hNTSR 1 or empty pCMV) and 0.6 µl Lipofectamine 2000 per well for 5 h. IP assays were performed 48 h after the transfection was started.

One day after the transfection, HEK293 cells were incubated in cell culture medium containing 5 µCi/ml myo-[2-³H]inositol. Following 24 h incubation, the cells were washed in HBSS (Gibco) and incubated in 100 µl per well HBSS containing 10 mM LiCl for 30 min at 37°C. The ligands were added and the cells incubated for 45 min at 37° C. The cells were then lysed in 10 mM formic acid for > 40 min. 20 µl of the lysate were transferred to a white 96-well plate containing 80 µl of yttrium silicate Scintillation Proximity Assay (YSi-SPA) beads (PerkinElmer). Lyophilized YSi-SPA beads were reconstituted in H₂O (1 g in 10 ml) and then diluted 1:8 before use. Plates were sealed, shaken at maximum speed for 10 min and centrifuged at 400 g for 5 min. After an 8 h delay, γ-radiation was measured in a Packard Top Count NXT scintillation plate reader. Determinations were made in duplicate or triplicate. As shown in Fig.1B and Table 1, myo-[2-³H]inositol accumulates similarly in the GLP-1/NT fusion peptide (SEQ ID NO: 45) sample as it does in the NT peptide sample, indicating that the GLP-1/NT fusion peptide acts on the NTSR1 receptor with similar potency to NT.

**Table 1**

| | | NT | GLP1-NT |
|---|---|---|---|
| Sigmoidal dose-response (variable slope) | | | |
| Best-fit values | | | |
| | Bottom | 176 | 139.4 |
| | Top | 436.1 | 333.6 |
| | LogEC50 | -8.603 | -8.502 |
| | HillSlope | 1.11 | 2.019 |
| | EC50 | 2.494e-009 | 3.149e-009 |

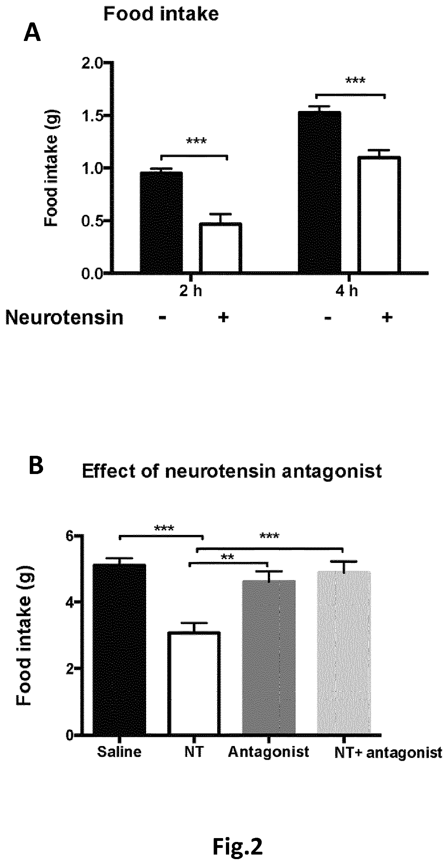

Example 3: Fig. 2 shows the effect of NT alone and NT+NT antagonist on food intake. Graph A shows the effect of saline or NT 3600 nmol/kg on acute 2h and 4h food intake in lean mice. Graph B shows the effect of saline, NT 150 nmol/kg, NTS1/NTS2 antagonist SR142948A 692 nmol/kg or NT+SR142948A on acute 30 min food intake in lean rats. Chow fed mice/rats fasted overnight were injected intraperitoneally in the beginning of the light cycle and food returned (n=8-12/group). Food intake was measured continuously after injection in an indirect calorimetry system. Data from both graphs was analyzed using a one-way ANOVA analysis followed by a Tukey's post hoc test. Mean ± SEM depicted on graphs. **p<0.01 and ***p<0.001 between groups.

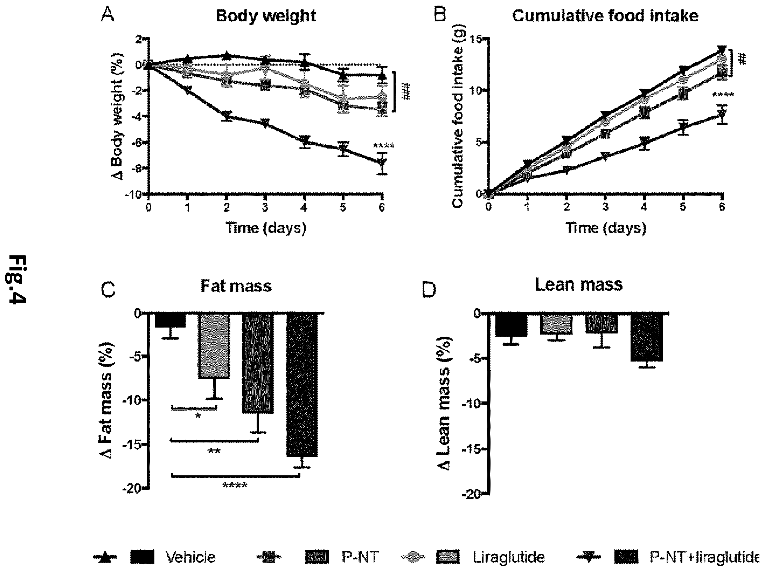

Example 4: As shown in fig.4, P-NT+liraglutide has a significantly greater effect on reducing body weight, food intake and lean or fat body mass in DIO mice relative to either liraglutide or P-NT alone.

DIO mice fed a high fat high sucrose diet (58% kcal from fat) (for over 4 months before study initiation) were injected subcutaneously right before lights out daily for 6 days, and food intake (FI) and body weight (BW) measured daily (n=6/group). Food intake and body weight were analysed using repeated measures two way ANOVA (A+B) or one-way ANOVA analysis (C+D), both followed by a Tukey's post hoc test. Mean ± SEM depicted on graphs. Graph A+B) ^{##}p<0.01, ^{###}p<0.001 and ****p<0.0001 combination treatment versus all other treatment groups. Graphs C+D) *p<0.05, **p<0.01 and ****p<0.0001 between groups.

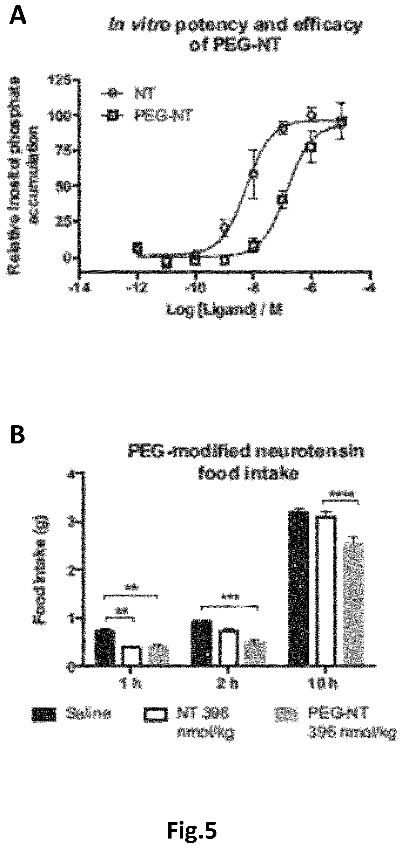

Example 5: As shown in fig.5, Peg-NT has a greater in-vitro potency and efficacy in an inositol phosphate accumulation assay and in-vivo on food intake, relative to NT. Graph A shows the accumulation of intracellular IP3 upon stimulation with increasing concentrations of Peg-NT in HEK293 cells transfected with the NTS1 receptor. Data has been normalized to maximal NT response (100%) and to vector (pcDNA) response (0%). Graph B shows the effect of NT or Peg-NT on acute food intake in lean mice. Chow fed lean mice fasted overnight were injected subcutaneously in the beginning of the light cycle and food returned. Food intake was measured continuously after injection in an indirect calorimetry system (n = 7-8/group). Food intake was analyzed using a one-way ANOVA analysis followed by a Tukey's post hoc test. Mean ± SEM depicted on graphs. **p<0.01, ***p<0.001 and ****p<0.0001 between groups.

Example 6: As shown in table 2 below, P-NT+liraglutide has a greater effect on reducing cholesterol, leptins, insulin, and glucose blood levels in DIO mice relative to either liraglutide or P-NT alone.

DIO mice fed a high fat high sucrose diet (58% kcal from fat) (for over 4 months before study initiation) were injected subcutaneously right before lights out daily for 6 days (n=6/group). On the day of termination, mice were fasted for 4 h before blood sampling (tail vein) and blood glucose measurement (glucometer) were performed. Blood biochemistry markers were analysed using a one-way ANOVA followed by a Tukey's post hoc test.

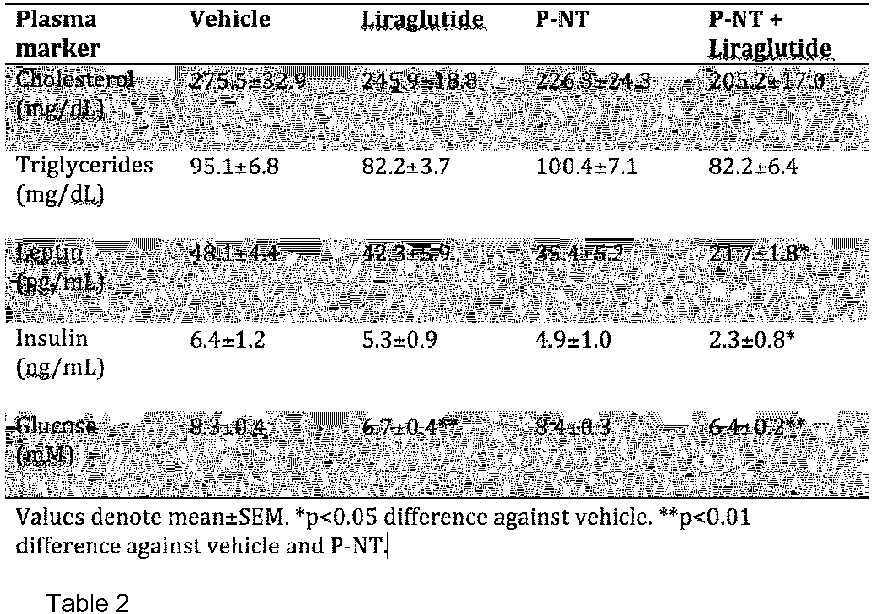

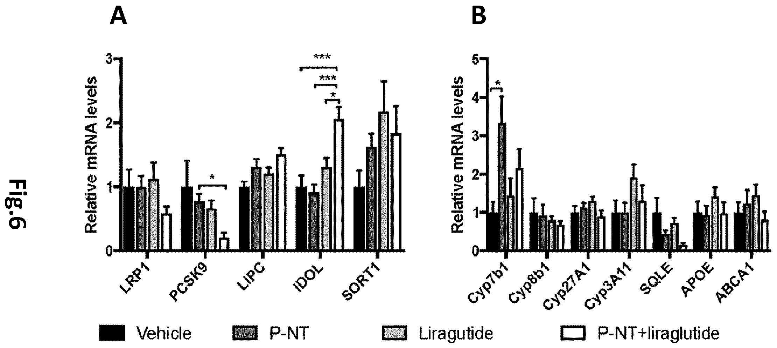

Example 7: Fig. 6 shows the effect of 6 day NT (396 nmol/kg), liraglutide (8 nmol/kg) or NT+liraglutide treatment on selected hepatic gene expression levels in DIO mice. DIO mice fed a high fat high sucrose diet (58% kcal from fat) (for over 4 months before study initiation) were injected subcutaneously right before lights out daily for 6 days (n=6/group). On the day of termination, mice were fasted for 4 hours before receiving a final injection of peptides. 2h after the final injection mice were sacrificed and liver samples collected. Gene expression levels were analysed using a one-way ANOVA followed by a Tukey's post hoc test. Mean ± SEM depicted on graphs. *p<0.05 and ***p<0.001 between groups.

Example 8: The following table 3 shows different synthesized peptides of the invention. More particularly, the peptides shown are shortened GLP-1-NT combination peptides ± a KKGG linker.

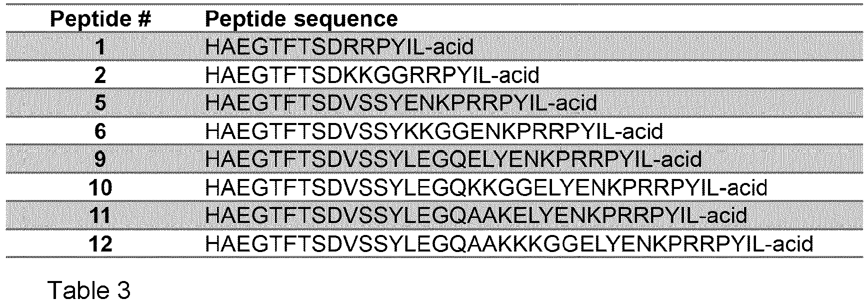

The peptides of this table are among the peptides referred to in the following examples.

Example 9: As shown in fig.7, shortened GLP-1-NT combination peptides reduces food intake in lean mice in comparison to saline in chow fed lean mice.

Chow fed lean mice fasted overnight were injected once SC in the beginning of the light cycle and food returned. Food intake measured continuously after injection in an indirect calorimetry system. Food intake was analysed using repeated measures two way ANOVA followed by a Tukey's post hoc test. A one-way ANOVA followed by a Tukey's post hoc test was performed in the inset in graph B. *p<0.05 peptide 9 vs. saline, ^{#}p<0.05 peptide 10 vs. saline, ^{##}p<0.01 peptide 10 vs. saline, ^{###}p<0.001 peptide 10 vs. saline.

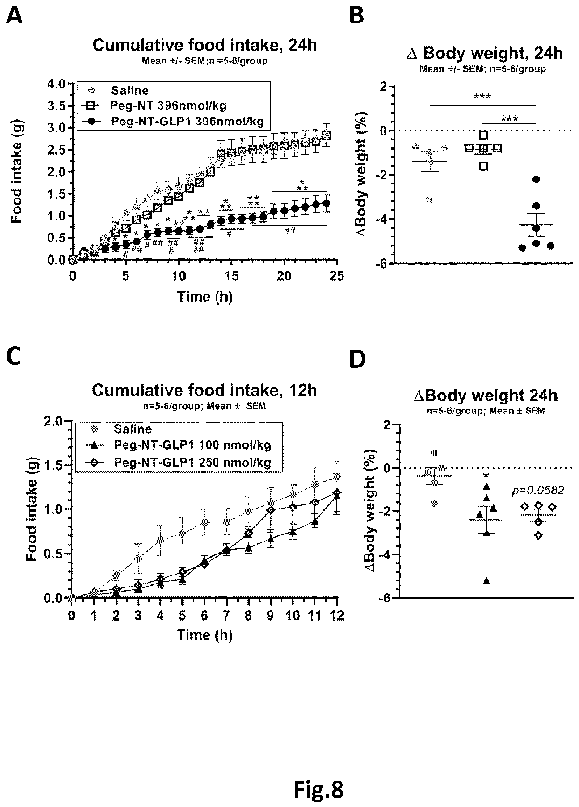

Example 10: As shown in fig.8, a full Peg-NT-GLP-1 (100 nmol/kg) combination peptide at different dose levels reduces acute food intake and body weight in DIO mice relative to both saline and Peg-NT (100 nmol/kg) after sub-chronic treatment in DIO mice.

Non-fasted DIO mice fed a high-fat high sucrose diet (58% kcal from fat) (for over 4 months before study initiation) were injected with a single dose subcutaneously right before light outs and food intake measured continuously in an indirect calorimetry system (A-B: mean BW±SEM 57.4±1.1 g at study initiation; C-D: mean BW±SEM 48.9±1.5 g at study initiation).

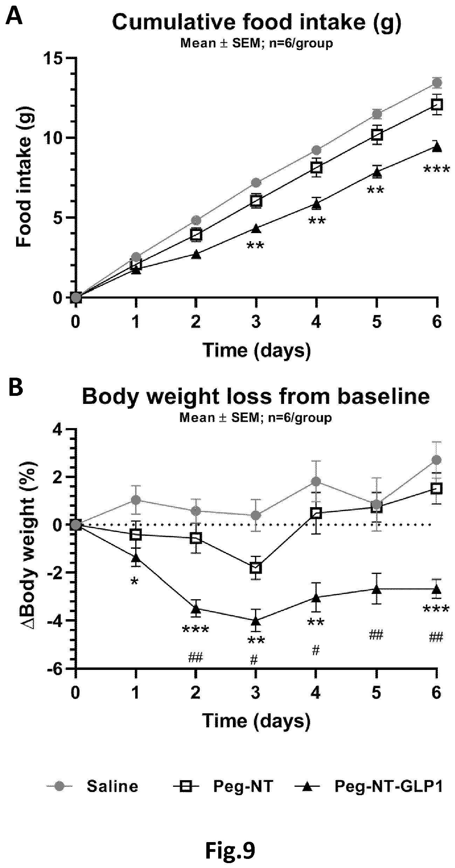

Example 11: As shown in fig.9, a full Peg-NT-GLP-1 (100 nmol/kg) combination peptides reduces cumulative food intake and induces body weight loss relative to both saline and Peg-NT (100 nmol/kg) after sub-chronic treatment in DIO mice.

Non-fasted DIO mice fed a high-fat high sucrose diet (58% kcal from fat) (for over 4 months before study initiation) were injected with a single dose subcutaneously right before light outs and food intake measured continuously in an indirect calorimetry system (A-B: mean BW±SEM 57.4±1.1 g at study initiation; C-D: mean BW±SEM 48.9±1.5 g at study initiation). Food intake was analysed using repeated measures two way ANOVA (A+C) or one-way ANOVA (B+D) analysis, both followed by a Tukey's post hoc test. Graph A) *p<0.05 saline vs. Peg-NT-GLP1, **p<0.01 saline vs. Peg-NT-GLP1, ***p<0.001 saline vs. Peg-NT-GLP1, ****p<0.0001 saline vs. Peg-NT-GLP1, ^{#}p<0.05 Peg-NT vs. Peg-NT-GLP1, ^{##}p<0.01 Peg-NT vs. Peg-NT-GLP1, ^{###}p<0.001 Peg-NT vs. Peg-NT-GLP1, ^{####}p<0.0001 Peg-NT vs. Peg-NT-GLP1. Graphs B and D) *p<0.05 vs. saline, ***p<0.001 vs. saline.

### Conclusion:

The above embodiments collectively show that the combination of NT and GLP1 analogues reduces acute food intake in mice to a higher degree than monotherapy treatment. Surprisingly, NT and GLP1 combination peptides had an even greater effect on reducing acute food intake in mice than a loose combination of the same. This was unexpected as common knowledge in the field dictates that a loose combination of two peptides should generally have a superior bioavailability for their two sites of actions, than a fusion peptide of the same two peptides. Further, pegylation of NT (Peg-NT) induces a synergistic effect with GLP-1 analogues (in a loose combination or in a full combination peptide) on food intake, body weight and adiposity in sub-chronic treatment settings. Peg-NT and liraglutide combination treatment also gives evidence of inducing a beneficial effect on glycemia and hepatic lipid handling and removal of blood cholesterol. The combination NT and GLP-1 and/or NT and GLP-1 analogues thus represents a novel and promising treatment for obesity and possibly co-morbidities.

## Claims

1. A fusion peptide comprising a first peptide linked to a second peptide, which first peptide comprises the sequence:
X₁-X₂-X₃-X₄-X₅-X₆-X₇-X₈-X₉
wherein
X₁ is L-histidine, D-histidine, desamino-histidine, 2-amino-histidine, β hydroxy-histidine, homohistidine, N^{α}-acetyl-histidine, α-fluoromethyl-histidine, α-methyl-histidine, 3-pyridylalanine, 2-pyridylalanine or 4-pyridylalanine;
X₂ is A, G, V, L, I, K, S, aminoisobutyric acid (Aib), (1-aminocyclopropyl) carboxylic acid, (1 aminocyclobutyl) carboxylic acid, (1-aminocyclopentyl) carboxylic acid, (1 aminocyclohexyl) carboxylic acid, (1-aminocycloheptyl) carboxylic acid, or (1-aminocyclooctyl) carboxylic acid;
X₃ is E, D or Q;
X₄ is G or A;
X₅ is T, V, S or I;
X₆ is F or Y;
X₇ is T, or S;
X₈ is S, V, or D;
X₉ is S, D, E, N or is not present, or
the first peptide comprising the sequence laid out in SEQ ID NO: 40 or SEQ ID NO: 41 or SEQ ID NO: 42, and
which second peptide has an amino acid sequence with at least 70% identity with any one of SEQ ID NO:24 to SEQ ID NO:31, or wherein the second peptide is selected from the list consisting of:
X₁₀-X₁₁-P-X₁₂-I-L;
P-X₁₀-X₁₁-P-X₁₂-I-L;
K-P-X₁₀-X₁₁-P-X₁₂-I-L;
N-K-P-X₁₀-X₁₁-P-X₁₂-I-L;
E-N-K-P-X₁₀-X₁₁-P-X₁₂-I-L;
Y-E-N-K-P-X₁₀-X₁₁-P-X₁₂-I-L;
L-Y-E-N-K-P-X₁₀-X₁₁-P-X₁₂-I-L;
Q-L-V-E-N-K-P-X₁₀-X₁₁-P-X₁₂-I-L; or
E-L-Y-E-N-K-P-X₁₀-X₁₁-P-X₁₂-I-L
wherein
X₁₀ is R or K;
X₁₁ is R or K; and
X₁₂ is Y, S, C or T, and wherein the fusion peptide is a dual agonist of both a glucagon like peptide 1 receptor and a neurotensin receptor.

2. The fusion peptide according to claim 1 comprising a first peptide linked to a second peptide, which first peptide comprises the sequence:
X₁-X₂-X₃-X₄-X₅-X₆-X₇-X₈-X₉-X₁₀
wherein
X₁ is L-histidine, D-histidine, desamino-histidine, 2-amino-histidine, β hydroxy-histidine, homohistidine, N^{α}-acetyl-histidine, α-fluoromethyl-histidine, α-methyl-histidine, 3-pyridylalanine, 2-pyridylalanine or 4-pyridylalanine;
X₂ is A, G, V, L, I, K, S, aminoisobutyric acid (Aib), (1-aminocyclopropyl) carboxylic acid, (1 aminocyclobutyl) carboxylic acid, (1-aminocyclopentyl) carboxylic acid, (1 aminocyclohexyl) carboxylic acid, (1-aminocycloheptyl) carboxylic acid, or (1-aminocyclooctyl) carboxylic acid;
X₃ is E, D or Q;
X₄ is G or A;
X₅ is T, V, S or I;
X₆ is F or Y;
X₇ is T, or S;
X₈ is S, V, or D;
X₉ is S, D, E, N or is not present;
X₁₀ is V or
the first peptide comprising the sequence laid out in SEQ ID NO: 40 or SEQ ID NO: 41 or SEQ ID NO: 42.

3. The fusion peptide according to any preceding claims, wherein the first peptide has at least 70% identity with any one of SEQ ID NO:2 to SEQ ID NO: 23 or SEQ ID NO: 34 to SEQ ID NO: 39 and/or wherein the second peptide is any of the sequences laid out in SEQ ID NO: 24-30.

4. The fusion peptide according to any preceding claims, wherein the first peptide defined in claim 1 is the N-terminus of the fusion peptide.

5. The fusion peptide according to any preceding claims, wherein the C-terminus of the fusion peptide has the amino acid sequence laid out in SEQ ID NO:30.

6. The fusion peptide according to claim 1, wherein the fusion peptide is a peptide having at least 70% identity with SEQ ID NO:32 or wherein the fusion peptide is a peptide having at least 70% identity with SEQ ID NO:33.

7. The fusion peptide according to any one of the preceding claims, wherein the first peptide is linked to the second peptide via a linker molecule.

8. The fusion peptide according to any one of the preceding claims, wherein the first peptide is H-A-E-G-T-F-T-S-D-V-S-S-Y-L-E-G-Q (SEQ ID No: 15), and optionally wherein the second peptide is E-L-Y-E-N-K-P-R-R-P-Y-I-L.

9. The fusion peptide according to claim 1, wherein the first peptide has the sequence H-A-E-G-T-F-T-S-D-V-S-S-Y-L-E-G-Q-A-A-K-E-F-I-A-W-L-V-K-G-R (SEQ ID No:2) and the second peptide is E-L-Y-E-N-K-P-R-R-P-Y-I-L.

10. The fusion peptide according to any one of the preceding claims, wherein the C-terminal end of said fusion peptide is amidated and/or wherein the fusion peptide is pegylated.

11. The fusion peptide according to any of the claims 1-9, wherein the fusion peptide is linked to an albumin binding moiety via a spacer, and optionally wherein the albumin binding moiety linked via a spacer is attached to said fusion peptide via the ε-amino group of a lysine residue.

12. A nucleic acid molecule having a sequence encoding a fusion peptide according to any one of claims 1 to 9, wherein the fusion peptide is a polypeptide.

13. A vector comprising the nucleic acid molecule according to claim 12.

14. A host cell comprising the nucleic acid molecule according to claim 12 or the vector according to claim 13.

15. A pharmaceutical composition comprising the fusion peptide according to any one of claims 1 to 9 and a pharmaceutically acceptable carrier.

16. The fusion peptide according to any one of claims 1 to 9 for use in therapy, such as for treatment or prophylaxis of diseases of the liver or cardiovascular diseases.

## Patentansprüche

1. Fusionspeptid, das ein mit einem zweiten Peptid verbundenes erstes Peptid umfasst, wobei das erste Peptid die Sequenz:
X₁-X₂-X₃-X₄-X₅-X₆-X₇-X₈-X₉
umfasst, wobei
X₁ L-Histidin, D-Histidin, Desaminohistidin, 2-Aminohistidin, (β-Hydroxyhistidin, Homohistidin, N^{α}-Acetylhistidin, α-Fluormethylhistidin, α-Methylhistidin, 3-Pyridylalanin, 2-Pyridylalanin oder 4-Pyridylalanin ist;
X₂ A, G, V, L, I, K, S, Aminoisobuttersäure (Aib), (1-Aminocyclopropyl)carbonsäure, (1 Aminocyclobutyl)-carbonsäure, (1-Aminocyclopentyl)carbonsäure, (1 Aminocyclohexyl)carbonsäure, (1-Aminocycloheptyl)-carbonsäure oder (1-Aminocyclooctyl)carbonsäure ist;
X₃ E, D oder Q ist;
X₄ G oder A ist;
X₅ T, V, S oder I ist;
X₆ F oder Y ist;
X₇ T oder S ist;
X₈ S, V oder D ist;
X₉ S, D, E, N ist oder nicht vorhanden ist oder das erste Peptid die in SEQ ID Nr.: 40 oder SEQ ID Nr.: 41 oder SEQ ID Nr.: 42 dargelegte Sequenz umfasst und wobei das zweite Peptid eine Aminosäuresequenz mit einer Sequenzidentität von mindestens 70 % mit einer beliebigen von SEQ ID Nr.: 24 bis SEQ ID Nr.: 31 aufweist oder wobei das zweite Peptid ausgewählt ist aus der Liste bestehend aus:
X₁₀-X₁₁-P-X₁₂-I-L;
P-X₁₀-X₁₁-P-X₁₂-I-L;
K-P-X₁₀-X₁₁-P-X₁₂-I-L;
N-K-P-X₁₀-X₁₁-P-X₁₂-I-L;
E-N-K-P-X₁₀-X₁₁-P-X₁₂-I-L;
Y-E-N-K-P-X₁₀-X₁₁-P-X₁₂-I-L;
L-Y-E-N-K-P-X₁₀-X₁₁-P-X₁₂-I-L;
Q-L-Y-E-N-K-P-X₁₀-X₁₁-P-X₁₂-I-L oder
E-L-Y-E-N-K-P-X₁₀-X₁₁-P-X₁₂-I-L,
wobei
X₁₀ R oder K ist;
X₁₁ R oder K ist und
X₁₂ Y, S, C oder T ist und wobei das Fusionspeptid ein Doppelagonist sowohl eines Glucagon-like-Peptid-1-Rezeptors als auch eines Neurotensin-Rezeptors ist.

2. Fusionspeptid nach Anspruch 1, das ein mit einem zweiten Peptid verbundenes erstes Peptid umfasst, wobei das erste Peptid die Sequenz:
X₁-X₂-X₃-X₄-X₅-X₆-X₇-X₈-X₉-X₁₀
aufweist, wobei
X₁ L-Histidin, D-Histidin, Desaminohistidin, 2-Aminohistidin, (β-Hydroxyhistidin, Homohistidin, N^{α}-Acetylhistidin, α-Fluormethylhistidin, α-Methylhistidin, 3-Pyridylalanin, 2-Pyridylalanin oder 4-Pyridylalanin ist;
X₂ A, G, V, L, I, K, S, Aminoisobuttersäure (Aib), (1-Aminocyclopropyl)carbonsäure, (1 Aminocyclobutyl)-carbonsäure, (1-Aminocyclopentyl)carbonsäure, (1 Aminocyclohexyl)carbonsäure, (1-Aminocycloheptyl)-carbonsäure oder (1-Aminocyclooctyl)carbonsäure ist;
X₃ E, D oder Q ist;
X₄ G oder A ist;
X₅ T, V, S oder I ist;
X₆ F oder Y ist;
X₇ T oder S ist;
X₈ S, V oder D ist;
X₉ S, D, E, N ist oder nicht vorhanden ist;
X₁₀ V ist oder
das erste Peptid die in SEQ ID Nr.: 40 oder SEQ ID Nr.: 41 oder SEQ ID Nr.: 42 dargelegte Sequenz umfasst.

3. Fusionspeptid nach einem der vorhergehenden Ansprüche, wobei das erste Peptid eine Identität von mindestens 70 % mit einem beliebigen von SEQ ID Nr.: 2 bis SEQ ID Nr.: 23 oder SEQ ID Nr.: 34 bis SEQ ID Nr.: 39 aufweist und/oder wobei das zweite Peptid eine beliebige der in SEQ ID Nr.: 24-30 dargelegten Sequenzen ist.

4. Fusionspeptid nach einem der vorhergehenden Ansprüche, wobei das in Anspruch 1 definierte erste Peptid der N-Terminus des Fusionspeptids ist.

5. Fusionspeptid nach einem der vorhergehenden Ansprüche, wobei der C-Terminus des Fusionspeptids die in SEQ ID Nr.: 30 dargelegte Aminosäuresequenz aufweist.

6. Fusionspeptid nach Anspruch 1, wobei das Fusionspeptid ein Peptid mit einer Identität von mindestens 70 % mit SEQ ID Nr.: 32 ist oder wobei das Fusionspeptid ein Peptid mit einer Identität von mindestens 70 % mit SEQ ID Nr.: 33 ist.

7. Fusionspeptid nach einem der vorhergehenden Ansprüche, wobei das erste Peptid über ein Linkermolekül mit dem zweiten Peptid verbunden ist.

8. Fusionspeptid nach einem der vorhergehenden Ansprüche, wobei das erste Peptid H-A-E-G-T-F-T-S-D-V-S-S-Y-L-E-G-Q (SEQ ID Nr.: 15) ist und wobei das zweite Peptid gegebenenfalls E-L-Y-E-N-K-P-R-R-P-Y-I-L ist.

9. Fusionspeptid nach Anspruch 1, wobei das erste Peptid die Sequenz H-A-E-G-T-F-T-S-D-V-S-S-Y-L-E-G-Q-A-A-K-E-F-I-A-W-L-V-K-G-R (SEQ ID Nr.: 2) aufweist und das zweite Peptid E-L-Y-E-N-K-P-R-R-P-Y-I-L ist.

10. Fusionspeptid nach einem der vorhergehenden Ansprüche, wobei das C-terminale Ende des Fusionspeptids amidiert ist und/oder wobei das Fusionspeptid pegyliert ist.

11. Fusionspeptid nach einem der Ansprüche 1-9, wobei das Fusionspeptid über einen Spacer mit einem albuminbindenden Rest verbunden ist und wobei der über einen Spacer verbundene albuminbindende Rest gegebenenfalls über die ε-Aminogruppe eines Lysinrests am Fusionsprotein gebunden ist.

12. Nukleinsäuremolekül mit einer Sequenz, die für ein Fusionsprotein nach einem der Ansprüche 1 bis 9 codiert, wobei das Fusionspeptid ein Polypeptid ist.

13. Vektor, der das Nukleinsäuremolekül nach Anspruch 12 umfasst.

14. Wirtszelle, die das Nukleinsäuremolekül nach Anspruch 12 oder den Vektor nach Anspruch 13 umfasst.

15. Pharmazeutische Zusammensetzung, die das Fusionsprotein nach einem der Ansprüche 1-9 und einen pharmazeutisch annehmbaren Träger umfasst.

16. Fusionsprotein nach einem der Ansprüche 1 bis 9 zur Verwendung in einer Therapie, wie zur Behandlung oder Prophylaxe von Krankheiten der Leber oder kardiovaskulären Krankheiten.

## Revendications

1. Peptide de fusion comprenant un premier peptide lié à un deuxième peptide, ledit premier peptide comprenant la séquence :
X₁-X₂-X₃-X₄-X₅-X₆-X₇-X₈-X₉
dans laquelle
X₁ est L-histidine, D-histidine, désamino-histidine, 2-amino-histidine, β-hydroxy-histidine, homohistidine, N^{α}-acétyl-histidine, α-fluorométhyl-histidine, α-méthyl-histidine, 3-pyridylalanine, 2-pyridylalanine ou 4-pyridylalanine ;
X₂ est A, G, V, L, I, K, S, l'acide aminoisobutyrique (Aib), l'acide (1-aminocyclopropyl)carboxylique, l'acide (1-aminocyclobutyl)carboxylique, l'acide (1-aminocyclopentyl)carboxylique, l'acide (1-aminocyclohexyl)carboxylique, l'acide (1-aminocycloheptyl)carboxylique ou l'acide (1-aminocyclooctyl)carboxylique ;
X₃ est E, D ou Q ;
X₄ est G ou A ;
X₅ est T, V, S ou I ;
X₆ est F ou Y ;
X₇ est T ou S ;
X₈ est S, V ou D ;
X₉ est S, D, E, N ou n'est pas présent, ou
le premier peptide comprenant la séquence décrite dans SEQ ID NO : 40 ou SEQ ID NO : 41 ou SEQ ID NO : 42, et
ledit deuxième peptide ayant une séquence d'acides aminés avec au moins 70 % d'identité avec l'un quelconque de SEQ ID NO : 24 à SEQ ID NO : 31, ou dans lequel le deuxième peptide est choisi dans la liste constituée de :
X₁₀-X₁₁-P-X₁₂-I-L ;
P-X₁₀-X₁₁-P-X₁₂-I-L ;
K-P-X₁₀-X₁₁-P-X₁₂-I-L ;
N-K-P-X₁₀-X₁₁-P-X₁₂-I-L ;
E-N-K-P-X₁₀-X₁₁-P-X₁₂-I-L ;
Y-E-N-K-P-X₁₀-X₁₁-P-X₁₂-I-L ;
L-Y-E-N-K-P-X₁₀-X₁₁-P-X₁₂-I-L ;
Q-L-Y-E-N-K-P-X₁₀-X₁₁-P-X₁₂-I-L ; ou
E-L-Y-E-N-K-P-X₁₀-X₁₁-P-X₁₂-I-L
dans laquelle
X₁₀ est R ou K ;
X₁₁ est R ou K ; et
X₁₂ est Y, S, C ou T, et le peptide de fusion étant un agoniste double d'un récepteur de glucagon-like peptide 1 et d'un récepteur de neurotensine.

2. Peptide de fusion selon la revendication 1 comprenant un premier peptide lié à un deuxième peptide, ledit premier peptide comprend la séquence :
X₁-X₂-X₃-X₄-X₅-X₆-X₇-X₈-X₉-X₁₀
dans laquelle
X₁ est L-histidine, D-histidine, désamino-histidine, 2-amino-histidine, β-hydroxy-histidine, homohistidine, N^{α}-acétyl-histidine, α-fluorométhyl-histidine, α-méthyl-histidine, 3-pyridylalanine, 2-pyridylalanine ou 4-pyridylalanine ;
X₂ est A, G, V, L, I, K, S, l'acide aminoisobutyrique (Aib), l'acide (1-aminocyclopropyl)carboxylique, l'acide (1-aminocyclobutyl)carboxylique, l'acide (1-aminocyclopentyl)carboxylique, l'acide (1-aminocyclohexyl)carboxylique, l'acide (1-aminocycloheptyl)carboxylique ou l'acide (1-aminocyclooctyl)carboxylique ;
X₃ est E, D ou Q ;
X₄ est G ou A ;
X₅ est T, V, S ou I ;
X₆ est F ou Y ;
X₇ est T, ou S ;
X₈ est S, V ou D ;
X₉ est S, D, E, N ou n'est pas présent ;
X₁₀ est V ou
le premier peptide comprenant la séquence décrite dans SEQ ID NO : 40 ou SEQ ID NO : 41 ou SEQ ID NO : 42.

3. Peptide de fusion selon l'une quelconque des revendications précédentes, dans lequel le premier peptide présente au moins 70 % d'identité avec l'un quelconque de SEQ ID NO : 2 à SEQ ID NO : 23 ou SEQ ID NO : 34 à SEQ ID NO : 39 et/ou dans lequel le deuxième peptide est l'une quelconque des séquences décrites dans SEQ ID NO : 24 à 30.

4. Peptide de fusion selon l'une quelconque des revendications précédentes, dans lequel le premier peptide défini dans la revendication 1 est l'extrémité N-terminale du peptide de fusion.

5. Peptide de fusion selon l'une quelconque des revendications précédentes, dans lequel l'extrémité C-terminale du peptide de fusion a la séquence d'acides aminés décrite dans SEQ ID NO : 30.

6. Peptide de fusion selon la revendication 1, le peptide de fusion étant un peptide présentant au moins 70 % d'identité avec SEQ ID NO : 32 ou dans lequel le peptide de fusion est un peptide ayant au moins 70 % d'identité avec SEQ ID NO : 33.

7. Peptide de fusion selon l'une quelconque des revendications précédentes, dans lequel le premier peptide est lié au deuxième peptide par l'intermédiaire d'une molécule de lieur.

8. Peptide de fusion selon l'une quelconque des revendications précédentes, dans lequel le premier peptide est H-A-E-G-T-F-T-S-D-V-S-S-Y-L-E-G-Q (SEQ ID NO : 15), et facultativement dans lequel le deuxième peptide est E-L-Y-E-N-K-P-R-R-P-Y-I-L.

9. Peptide de fusion selon la revendication 1, dans lequel le premier peptide a la séquence H-A-E-G-T-F-T-S-D-V-S-S-Y-L-E-G-Q-A-A-K-E-F-I-A-W-L-V-K-G-R (SEQ ID NO : 2) et le deuxième peptide est E-L-Y-E-N-K-P-R-R-P-Y-I-L.

10. Peptide de fusion selon l'une quelconque des revendications précédentes, l'extrémité C-terminale dudit peptide de fusion étant amidée et/ou le peptide de fusion étant pégylé.

11. Peptide de fusion selon l'une quelconque des revendications 1 à 9, le peptide de fusion étant lié à un fragment de liaison d'albumine par l'intermédiaire d'un espaceur, et facultativement dans lequel le fragment de liaison d'albumine lié par l'intermédiaire d'un espaceur est lié audit peptide de fusion par l'intermédiaire du groupe ε-amino d'un résidu lysine.

12. Molécule d'acide nucléique ayant une séquence codant pour un peptide de fusion selon l'une quelconque des revendications 1 à 9, le peptide de fusion étant un polypeptide.

13. Vecteur comprenant la molécule d'acide nucléique selon la revendication 12.

14. Cellule hôte comprenant la molécule d'acide nucléique selon la revendication 12 ou le vecteur selon la revendication 13.

15. Composition pharmaceutique comprenant le peptide de fusion selon l'une quelconque des revendications 1 à 9 et un véhicule pharmaceutiquement acceptable.

16. Peptide de fusion selon l'une quelconque des revendications 1 à 9 pour utilisation en thérapie, par exemple pour le traitement ou la prophylaxie de maladies du foie ou de maladies cardiovasculaires.
